# EUROPEAN PATENT SPECIFICATION

(11) **EP 4 025 176 B1**
(45) Date of publication and mention of the grant of the patent: **01.10.2025**
(21) Application number: 19765440.3
(22) Date of filing: 04.09.2019
(51) Int. Cl.: A61K 8/06, A61K 8/19, A61K 8/26, A61K 8/37, A61K 8/41, A61K 8/86, A61K 8/97, A61Q 15/00

(54) **ANTIPERSPIRANT ACTIVE EMULSION**
SCHWEISSHEMMENDE WIRKSAME EMULSION
EMULSION ACTIVE ANTIPERSPIRANTE

(43) Date of publication of application: 13.07.2022
(73) Proprietor: Beiersdorf AG, 22529 Hamburg (DE)
(72) Inventor: VOGT, Melanie, 23867 Sülfeld (DE); OELRICHS, Ilka, 25436 Tornesch (DE)
(74) Representative: Beiersdorf AG
(86) International application number: PCT/EP2019/073544
(87) International publication number: WO 2021/043394

(56) References cited:
- WO-A1-2006/012971
- WO-A1-2006/119981
- WO-A1-2015/102916
- WO-A2-2010/097205

## Description

The present invention belongs to the cosmetic field and relates to an emulsion which forms an antiperspirant stick.

Solid cosmetic antiperspirant sticks which are in a form of an oil in water emulsion are commonly known by the person skilled in art.

E.g. the document WO 2006/012971 A1 discloses low-residue antiperspirant sticks on the basis of an oil in water dispersion. The stick compositions disclosed comprise an oil phase, emulsifier, an aqueous phase and aluminum components, which act as antiperspirant active material.

Additionally WO2017/162443 A1 discloses cosmetic antiperspirant sticks in form of an oil in water dispersion with improved from stability.

Furthermore, WO 2012/080017 A2 discloses in the examples 1.1 to 1.7 various antiperspirant sticks on the basis of an oil in water dispersion.

Further prior art documents are WO2010/097205 A1, WO2006/119981 A1, WO2006/012871 A1 und WO 2015/102916 A1. However, none of the documents could direct the person skilled in art to the present invention.

The pure fact that the person skilled in art knows a few antiperspirant sticks in form of an oil in water dispersion shall not obscure the fact that these prior art sticks suffer a number of disadvantages. One particular disadvantage is directed to the mass production of such emulsion sticks. Conventionally, the sticks are prepared by melting all ingredients of the oil phase and mixing the ingredients of the aqueous phase. Both phases are mixed at elevated temperatures. Furthermore, in order to form an emulsion the mixture is homogenized and afterwards stored at elevated temperatures, e.g. 70°C, until the obtained bulk is filled into a mold for cooling and forming the stick. The mold is usually the final packaging of the product.

Specific problems arise from the fact that it often occurs that the homogenized bulk is stored for several hours before all of the bulk is filled into the packaging. Within that time it is often observed that agglomerated particles are formed within the bulk. These can also be described as lumps. Accordingly, it is often needed to filter the bulk before filling it into the packaging. As such measures increase the production costs due to the use and maintenance of the filters it is generally desirable to avoid the formation of agglomerated particles.

An analysis has indicated that such particles mainly contain aluminum compounds, oils and waxes. Consequently, by the formation of agglomerated particles the active content of the antiperspirant active aluminum compound is reduced. This may lead to a reduced antiperspirant efficiency in the final product. Or higher quantities of the antiperspirant active ingredient has to be included in the bulk which makes the product more expensive to produce.

Accordingly, it is the objective of the present invention to provide an emulsion which can be stored at elevated temperatures, in particular at 70°C, for a prolonged amount of time, e.g. 8 hours, which does not form agglomerated particles in the bulk. It was surprisingly found that these objectives can be addressed by the objects of the present invention.

The present invention is an antiperspirant oil in water emulsion comprising, calculated to the total weight of the emulsion,
a) At least one antiperspirant active aluminum compound;
b) Palmitamidopropyltrimonium chloride;
c) at least one lipid and/or wax component with a melting point of >50° C;
d) at least one oil which is liquid at 20° C;
e) at least one nonionic emulsifier with an HLB value of more than 7;
f) at least one nonionic emulsifier with an HLB value greater than 1.0 and less than or equal to 7.0; and
g) 30% to 50% by weight water,
characterized in that the emulsion is liquid at 70 °C and solid at 20°C, 1013 hPa and a relative humidity of 50%.

All the weight percentages (% by weight) given below are based, unless otherwise stated, on the total weight of the emulsion of the invention. If ratios of certain components are disclosed in the following description, these ratios refer, unless otherwise stated, to weight ratios of the components.

Unless otherwise stated, all tests and measurements were performed under "normal conditions". The term "normal conditions" refers to 20°C, 1013 hPa and a relative humidity of 50%.

The term "skin" refers solely to human skin.

It was surprisingly found that the emulsion of the present invention does not form clumps in the bulk when being stored at elevated temperatures for a prolonged amount of time, e.g. 4 hours, in particular 8 hours.

According to the present invention the emulsion comprises at least one antiperspirant active aluminum compound. According to the invention it is preferred if this aluminum compound is selected from the group consisting of aluminum chlorohydrate; aluminum sesquichlorohydrate; aluminum zirconium chlorohydrates, such as aluminum zirconium trichlorohydrate, aluminum zirconium tetrachlorohydrate, aluminum zirconium pentachlorohydrate, aluminum zirconium octachlorohydrate; and the aluminum zirconium chlorohydrate glycine complexes such as aluminum zirconium trichlorohydrex glycine, aluminum zirconium tetrachlorohydrex glycine, aluminum zirconium pentachlorohydrex glycine and aluminum zirconium octachlorohydrex glycine. It is in particular preferred if aluminum chlorohydrate and/or aluminum sesquichlorohydrate are contained as antiperspirant active aluminum compound.

Embodiments which are also preferred according to the invention are characterized in that only aluminum chlorohydrate and/or aluminum sesquichlorohydrate are contained as antiperspirant active aluminum compound.

The following description is valid for all embodiments of the invention.

It is preferred if the total quantity of the antiperspirant active aluminum compound is in the range from 5 to 30% by weight, more preferably 10 to 25% by weight and most preferably 12.5 to 22.5% by weight, calculated to the total weight of the emulsion.

It is particular preferred if aluminum chlorohydrate and/or aluminum sesquichlorohydrate are contained as antiperspirant active aluminum compound and the total quantity of aluminum chlorohydrate and/or aluminum sesquichlorohydrate is in the range from 5 to 30% by weight, more preferably 10 to 25% by weight and most preferably 12.5 to 22.5% by weight, calculated to the total weight of the emulsion.

It is of particular advantage if aluminum chlorohydrate is contained as antiperspirant active aluminum compound and the total quantity of aluminum chlorohydrate is in the range from 5 to 30% by weight, more preferably 10 to 25% by weight and most preferably 12.5 to 22.5% by weight, calculated to the total weight of the emulsion. Here, it is further preferred, if no further antiperspirant active aluminum compound is contained.

For the cases that the emulsion comprises aluminum chlorohydrate and aluminum sesquichlorohydrate it is further preferred, if the ratio by weight between the aluminum chlorohydrate and aluminum sesquichlorohydrate is preferably in the range from 2:1 to 50:1, more preferably from 3.5:1 to 30:1, still more preferably 4:1 to 24:1 and most preferable 5:1 to 20:1.

According to the invention the emulsion also comprises palmitamidopropyltrimonium chloride, whereby it is favored if the total quantity of palmitamidopropyltrimonium chloride is in the range from 0.1 to 5% by weight, more preferably 0.2 to 3% by weight, more preferably 0.3 to 2% by weight, more preferably 0.4 to 1.5% by weight and most preferably 0.5 to 1.2% by weight, calculated to the total weight of the emulsion.

According to the invention the emulsion comprises at least one lipid and/or wax component with a melting point of >50° C. Thereby, it is preferred if the total quantity of the lipid and/or wax components with a melting point of >50°C is in the range from 8 to 18% by weight, more preferably 10 to 15% by weight calculated to the total weight of the emulsion.

Generally, waxes are of solid to brittle consistency, coarse to finely crystalline, transparent to opaque, but not glass-like, and melt above 50° C without decomposition. Just a little above the melting point they are of low viscosity and exhibit a heavily temperature-dependent consistency and solubility.

According to the invention, preference is given, to natural vegetable waxes, e.g., candelilla wax, carnauba wax, japan wax, sugar cane wax, ouricoury wax, cork wax, sunflower wax, fruit waxes, such as orange waxes, lemon waxes, grapefruit wax, and animal waxes, e.g., beeswax, shellac wax and spermaceti. For the purposes of the invention, it may be particularly preferred to use hydrogenated or hardened waxes. Wax components which can be used are also chemically modified waxes, in particular, the hard waxes, such as, for example, montan ester waxes, hydrogenated jojoba waxes and sasol waxes. Synthetic waxes, which are likewise preferred according to the invention are polyalkylene waxes and polyethylene glycol waxes, C20-C40-dialkyl esters of dimer acids, C30-50-alkyl beeswax and alkyl and alkylaryl esters of dimer fatty acids.

A particularly preferred wax component is chosen from at least one ester of a saturated monohydric C16-C60-alcohol and a saturated C8-C36-monocarboxylic acid, as long as those esters have a melting point above 50°C. It is particularly preferred to choose the wax components from the group of esters of saturated, branched or unbranched alkanecarboxylic acids of chain length from 12 to 24 carbon atoms and the saturated, branched or unbranched alcohols of chain length from 16 to 50 carbon atoms which have a melting point of >50° C. It is most preferred if the emulsion comprises at least cetyl palmitate as wax and/or lipid component with a melting point of >50°C.

If an ester of saturated, branched or unbranched alkanecarboxylic acids of chain length from 12 to 24 carbon atoms and the saturated, branched or unbranched alcohols of chain length from 16 to 50 carbon atoms, which has a melting point of >50°C, is contained in the emulsion of the invention, it is further preferred if the total quantity of this ester is in the range from 3 to 9% by weight, more preferably 4.5 to 7.5% by weight an most preferably, 5 to 7% by weight, calculated to the total weight of the emulsion.

If cetyl palmitate is contained in the emulsion of the invention, it is further preferred if the total quantity of cetyl palmitate is in the range from 3 to 9% by weight, more preferably 4.5 to 7.5% by weight an most preferably, 5 to 7% by weight, calculated to the total weight of the emulsion.

Furthermore, it is preferred according to the invention if the emulsion also comprises C20-C40-alkyl stearate as wax component. If C20-C40-alkyl stearate is contained, it is further preferred if the total quantity of C20-C40-alkyl stearate is in the range from 0.2 to 3% by weight, more preferably 0.5 to 2% by weight and most preferably 0.75 to 1.5% by weight, calculated to the total weight of the emulsion.

Further preferred lipid and/or wax components are the triglycerides of saturated and optionally hydroxylated C12-30-fatty acids, such as hydrogenated triglyceride fats, in particular hydrogenated palm oil, hydrogenated coconut oil, hydrogenated castor oil, glyceryl tribehenate (tribehenin) and/or glyceryl tri-12-hydroxystearate, also synthetic complete esters of fatty acids and glycols or polyols having 2-6 carbon atoms so long as they have a melting point above 50° C, for example, preferably C18-C36 acid triglyceride (Syncrowax^{®} HGL-C).

According to the invention, hydrogenated castor oil, obtainable, e.g., as the commercial product Cutina^{®} HR, is a further particularly preferred wax component which has a melting point exceeding 50°C. In the case hydrogenated castor oil is contained in the emulsion of the invention, it is further preferred if the total quantity of hydrogenated castor oil in the emulsion is in the range from 3 to 9% by weight, more preferably 4.5 to 7.5% by weight an most preferably, 5 to 7% by weight, calculated to the total weight of the emulsion.

Further preferred lipid or wax components with a melting point of >50° C are the saturated linear C14-C36-carboxylic acids, in particular myristic acid, palmitic acid, stearic acid and behenic acid, and mixtures of these compounds, e.g., Syncrowax^{®} AW 1C(C18-C36-fatty acids) or Cutina^{®} FS 45 (palmitic and stearic acid). According to the invention saturated linear C14-C36-carboxylic acids are not considered as emulsifier. In the case the emulsion comprises one or more saturated linear C14-C36-carboxylic acids, the total quantity of those carboxylic acid is preferably in the range from 1 to 8% by weight, more preferably 2 to 6% by weight and most preferably 3 to 5% by weight, calculated to the total weight of the emulsion.

A particular preferred embodiment of the invention is characterized in that the emulsion comprises the following lipid or wax components with a melting point of >50°C:
- an ester of saturated, branched or unbranched alkanecarboxylic acids of chain length from 12 to 24 carbon atoms and the saturated, branched or unbranched alcohols of chain length from 16 to 50 carbon atoms, which has a melting point of >50°C, preferably in a total quantity from 3 to 9% by weight, more preferably 4.5 to 7.5% by weight an most preferably, 5 to 7% by weight;
- Hydrogenated castor oil, preferably in a total quantity from 3 to 9% by weight, more preferably 4.5 to 7.5% by weight an most preferably, 5 to 7% by weight; and
- One or more saturated linear C14-C36-carboxylic acids, preferably in a total quantity from 1 to 8% by weight, more preferably 2 to 6% by weight and most preferably 3 to 5% by weight;
wherein the % by weight are calculated to the total weight of the emulsion.

Within this embodiment it is even more preferred, if the emulsion comprises the following lipid or wax components with a melting point of >50°C:
- Cetyl palmitate, preferably in a total quantity from 3 to 9% by weight, more preferably 4.5 to 7.5% by weight an most preferably, 5 to 7% by weight;
- Hydrogenated castor oil, preferably in a total quantity from 3 to 9% by weight, more preferably 4.5 to 7.5% by weight an most preferably, 5 to 7% by weight; and
- One or more saturated linear C14-C36-carboxylic acids, preferably in a total quantity from 1 to 8% by weight, more preferably 2 to 6% by weight and most preferably 3 to 5% by weight;
wherein the % by weight are calculated to the total weight of the emulsion.

Within this embodiment it is most preferred, if the emulsion comprises the following lipid or wax components with a melting point of >50°C:
- Cetyl palmitate, preferably in a total quantity from 3 to 9% by weight, more preferably 4.5 to 7.5% by weight an most preferably, 5 to 7% by weight;
- Hydrogenated castor oil, preferably in a total quantity from 3 to 9% by weight, more preferably 4.5 to 7.5% by weight an most preferably, 5 to 7% by weight;
- One or more saturated linear C14-C36-carboxylic acids, preferably in a total quantity from 1 to 8% by weight, more preferably 2 to 6% by weight and most preferably 3 to 5% by weight; and
- C20-C40-alkyl stearate, preferably in the range from 0.2 to 3% by weight, more preferably 0.5 to 2% by weight and most preferably 0.75 to 1.5% by weight;
wherein the % by weight are calculated to the total weight of the emulsion.

Furthermore, the emulsion of the invention comprises at least one oil which is liquid at 20° C.

The oil is preferably not a fragrance component or an essential oil. Preferred oils are chosen from branched saturated or unsaturated fatty alcohols having 6-30 carbon atoms, which are liquid at 20°C. These alcohols are also often referred to as Guerbet alcohols since they are obtainable according to the Guerbet reaction.

Further oils d) preferred according to the invention are chosen from the triglycerides of linear or branched, saturated or unsaturated, optionally hydroxylated C8-30-fatty acids. The use of natural oils, e.g., soya oil, cotton seed oil, sunflower oil, palm oil, palm kernel oil, linseed oil, almond oil, castor oil, corn oil, olive oil, rapeseed oil, sesame oil, thistle oil, wheat germ oil, peach kernel oil and the liquid fractions of coconut oil and the like may be particularly suitable. Also suitable, however, are synthetic triglyceride oils, in particular capric/caprylic triglycerides.

Further oils d) preferred according to the invention are chosen from the dicarboxylic acid esters of linear or branched C2-C10-alkanols, in particular, diisopropyl adipate, di-n-butyl adipate, di(2-ethylhexyl) adipate, dioctyl adipate, diethyl/di-n-butyl/dioctyl sebacate, diisopropyl sebacate, dioctyl malate, dioctyl maleate, dicaprylyl maleate, diisooctyl succinate, di-2-ethylhexyl succinate and di(2-hexyldecyl) succinate.

Further oils d) preferred according to the invention are chosen from the addition products of from 1 to 5 propylene oxide units onto mono- or polyhydric C8-22-alkanols, such as octanol, decanol, decanediol, lauryl alcohol, myristyl alcohol and stearyl alcohol, e.g., PPG-2 myristyl ether and PPG-3 myristyl ether.

Further oils d) are preferably chosen from the esters of the linear or branched saturated or unsaturated fatty alcohols having 2-30 carbon atoms with linear or branched saturated or unsaturated fatty acids having 2-30 carbon atoms, which may be hydroxylated and which are liquid at 20°C. These include hexyldecyl stearate, hexyldecyl laurate, isodecyl neopentanoate, isononyl isononanoate, C12-15 alkyl benzoate, 2-ethylhexyl palmitate, 2-ethylhexyl stearate, isopropyl myristate, isopropyl palmitate, isopropyl stearate, isopropyl isostearate, isopropyl oleate, isooctyl stearate, isononyl stearate, isocetyl stearate, isononyl isononanoate, isotridecyl isononanoate, cetearyl isononanoate, 2-ethylhexyl laurate, 2-ethylhexyl isostearate, 2-ethylhexyl cocoate, 2-octyldodecyl palmitate, butyloctanoic acid-2-butyl octanoate, diisotridecyl acetate, n-butyl stearate, n-hexyl laurate, n-decyl oleate, oleyl oleate, oleyl erucate, erucyl oleate, erucyl erucate, ethylene glycol dioleate and dipalmitate.

Further preferred oils d) which can be used are chosen from the addition products of at least 6 ethylene oxide and/or propylene oxide units onto mono- or polyhydric C3-22-alkanols. Especially preferred are PPG-14 butyl ether and PPG-15 stearyl ether.

A particular preferred embodiment of the invention is characterized in that the emulsion comprises at least one oil selected from the addition products of 10 to 20 propylene oxide units onto mono- or polyhydric C3-22-alkanols, which are liquid at 20° C. Within this embodiment it is preferred if the oil d) is selected from PPG-14 butyl ether and PPG-15 stearyl ether.

In another embodiment of the invention the emulsion comprises at least one oil selected from the addition products of 10 to 20 propylene oxide units onto mono- or polyhydric C3-22-alkanols, which are liquid at 20° C, and/or esters of the linear or branched saturated or unsaturated fatty alcohols having 2-30 carbon atoms with linear or branched saturated or unsaturated fatty acids having 2-30 carbon atoms, which are liquid at 20°C. It is particular preferred if the oil d) is selected from PPG-14 butyl ether, PPG-15 stearyl ether and/or esters of the linear or branched saturated or unsaturated fatty alcohols having 2-30 carbon atoms with linear or branched saturated or unsaturated fatty acids having 2-30 carbon atoms, which are liquid at 20°C. It is even more preferred if PPG-14 butyl ether, PPG-15 stearyl ether and/or C12-15 alkyl benzoate are contained.

It is further preferred according to the invention if oils that are liquid at 20°C are contained in a total quantity in the range from 10 to 30% by weight, more preferably 15 to 27% by weight and most preferably 18 to 25% by weight, calculated to the total weight of the emulsion.

Accordingly it is also preferred if PPG-14 butyl ether, PPG-15 stearyl ether and/or C12-15 alkyl benzoate are contained in a total quantity in the range from 10 to 30% by weight, more preferably 15 to 27% by weight and most preferably 18 to 25% by weight, calculated to the total weight of the emulsion. Thereby it is further preferred if at least PPG-14 butyl ether and C12-15 alkyl benzoate are contained. If PPG-14 butyl ether and C12-15 alkyl benzoate are contained the ratio by weight between PPG-14 butyl ether and C12-15 alkyl benzoate is preferably in the range from 10:1 to 1:1, more preferably 6:1 to 1.5:1, and most preferably 3:1 to 2:1.

According to the invention the emulsion comprises at least one nonionic emulsifier with an HLB value of more than 7. These are emulsifiers generally known to the person skilled in the art, as listed, for example, in Kirk-Othmer, "Encyclopedia of Chemical Technology", 3rd edition, 1979, volume 8, page 913-916.

It is preferred according to the invention if the total quantity of the nonionic emulsifier with an HLB value of more than 7 is in the range from 0.5 to 3% by weight, more preferably 1 to 2.5% by weight, based on the total weight of the emulsion.

Particular preference is given to emulsifier e), which are C12-C18-alkanols having in each case 10-30 units of ethylene oxide per molecule, and mixtures of these substances. In particular preferred are ceteth-12, ceteth-20, ceteth-30, steareth-12, steareth-20, steareth-21, oleth-20, steareth-30, laureth-12 and beheneth-20. It is most preferred if at least Steareth-21 and/or Oleth-20 are contained as emulsifier having an HLB value of more than 7. Accordingly it is also preferred if the emulsion comprises steareth-21 and/or oleth-20 in a total quantity from 0.5 to 3% by weight, more preferably 1 to 2.5% by weight, based on the total weight of the emulsion.

In other embodiments it is preferred if the at least one emulsifier e) is characterized in that it has an HLB value in the range from 14 to 17, more preferably from 14.5 to 16.5 and most preferably from 15 to 16. Accordingly it is also preferred if the emulsion comprises at least one emulsifier e) having an HLB value in the range from 14 to 17, more preferably from 14.5 to 16.5 and most preferably from 15 to 16, in a total quantity from 0.5 to 3% by weight, more preferably 1 to 2.5% by weight, based on the total weight of the emulsion.

Furthermore, the emulsion comprises at least one nonionic emulsifier with an HLB value greater than 1.0 and less than or equal to 7.0. It is preferred according to the invention if the nonionic emulsifier f) has an HLB value in the range from 3 to 6.5, more preferably 4 to 6.

Those emulsifiers having an HLB value greater than 1.0 and less than or equal to 7.0, preferably from 3 to 6.5 and most preferably 4 to 6 are preferably contained in the emulsion in a total quantity from 0.1% to 1.5% by weight, more preferably 0.3 to 1% by weight, calculated to the total weight of the emulsion.

Preferred embodiments of the invention are characterized in that at least one nonionic emulsifier having a HLB value in the range from 3 to 6.5 is contained in a total quantity from 0.1% to 1.5% by weight, more preferably 0.3 to 1% by weight, calculated to the total weight of the emulsion.

Further preferred embodiments of the invention are characterized in that at least one nonionic emulsifier having a HLB value in the range from 4 to 6 is contained in a total quantity from 0.1% to 1.5% by weight, more preferably 0.3 to 1% by weight, calculated to the total weight of the emulsion.

A particular preferred nonionic emulsifier having an HLB value of 4.9 is Steareth-2. Accordingly it is preferred if the emulsion comprises at least Steareth-2 as nonionic emulsifier f). The quantity of Steareth-2 is preferably in the range from 0.1% to 1.5% by weight, more preferably 0.3 to 1% by weight, calculated to the total weight of the emulsion.

According to the invention it is further preferred if the emulsion comprises propylene glycol, whereby it is preferred if the total quantity of propylene glycol is in the range from 0.5 to 4% by weight calculated to the total weight of the emulsion.

Furthermore, it is preferred according to the invention, if the emulsion further comprises at least one fatty alcohol selected from the group consisting of cetyl alcohol and stearyl alcohol. Accordingly it is according to the invention if cetyl alcohol and/or stearyl alcohol are included. Mixtures of both ingredients are well known under the CTFA designation cetearyl alcohol.

In the case the emulsion comprises cetyl alcohol, stearyl alcohol and/or mixtures thereof it is preferred, if the total quantity of those alcohols is in the range from 1 to 5% and more preferably from 1.5% to 3.5% by weight calculated to the total weight of the emulsion.

According to the invention cetyl alcohol and/or stearyl alcohol are not considered as emulsifier e) or f) and also not as an oil or wax component c).

The water content of the emulsion is preferably in the range from 32 to 45% by weight calculated to the total weight of the emulsion.

Furthermore, the emulsion of the invention is characterized in that that the emulsion is liquid at 70° C and is solid under normal conditions.

After storing 24 hours under normal conditions the emulsion is preferably characterized in that it has a penetration force value in the range from 150-800 gram-force (g-force), preferably 200-750 gram-force (g-force), particularly preferably 350-600 gram-force (g-force), at a penetration depth of 5,000 mm. The penetration force value is a measure of the hardness of a stick (and also of a solid cream composition) and indicates at what maximum force a defined measuring probe, here a stainless steel 45° cone (model TA 15) is inserted perpendicularly (axially) into the antiperspirant mass to be measured at a feed rate of 2 mm/second to a penetration depth suitable for the measurement, here to a penetration depth of 5,000 mm. The determination of the penetration force value is carried out using the TA-XT2i texture analyzer from Stable Micro Systems (Vienna Court, Lammas Road, Godalming, Surrey GU7 1YL). The maximum force is given in gram-force (g-force). Here, lower values indicate a softer composition, harder compositions have a higher penetration force value. The measurements are carried out under normal conditions.

The present invention further provides a process for the preparation of an antiperspirant stick, wherein the wax and oil components c) and d) are heated together with the emulsifier e) and f) and optionally all further oil soluble ingredients to 90-95° C and melted. Then the water containing the water-soluble ingredients is likewise heated to 90-95° C is added with vigorous stirring, optionally further ingredients are mixed in. Afterwards the formed emulsion is stored at 70°C and poured into suitable dispenser molds and solidified by static cooling (without further stirring) to normal conditions.

Furthermore, it is preferred if the emulsion of the invention does not comprise any particles which are insoluble in water and which are solid at any temperature above 95°C.

### Examples:

The following examples should illustrate the compositions of this invention, without intending to limit the invention to these examples. The numerical values in the examples are percentages by weight, based on the total weight of the preparations.

| **Ingredient** | **Com. 1** | **Ex. 1** |
|---|---|---|
| Aluminum Chlorohydrate | 15 | 15 |
| Palmitamidopropyltrim onium Chloride | - | 0.6 |
| Cetyl Palmitate | 6 | 6 |
| Hydrogenated Castor Oil | 6 | 6 |
| PPG-14 Butyl Ether | 15 | 15 |
| C12-15 Alkyl Benzoate | 5.6 | 5.6 |
| C20-40 Alkyl Stearate | 1 | 1 |
| Steareth-21 | 0.5 | 0.5 |
| Steareth-2 | 0.5 | 0.5 |
| Oleth-20 | 1.0 | 1.0 |
| Fatty Acid mixture consisting of 47% Stearic Acid, 48.5% Palmitic Acid, 2.5% Myristic Acid, 1.5% Arachidic Acid and 0.5% Oleic Acid | 1.2 | 1.2 |
| Propylene Glycol | 2 | 2.4 |
| Cetearyl Alcohol | 2.5 | 2.5 |
| Parfum | q.s. | q.s. |
| Aqua | ad 100 | ad 100 |
| Stability of the emulsion after storing for 4 hours at 70°C | Lumps suspend ed in the | No lumps |
| | emulsio n | |

The examples above were prepared in the following manner:
a) Heat the oil phase including cetyl palmitate, hydrogenated castor oil, palmitamidopropyltrimonium chloride, PPG-14 butyl ether and C12-15 alkyl benzoate, steareth-21, steareth-2, oleth-20, palmitic acid + stearic acid + myristic acid + arachidic acid + oleic acid, cetearyl alcohol or cetyl alcohol and stearyl alcohol and C20-40 alkyl stearate up to 90°C.
b) Heat the water phase including aluminum chlorohydrate, propylene glycol and water up to 85°C.
c) Bring both phases together under homogenizing to build the emulsion and then cool down to 70°C and add the perfume and homogenize a second time.

Afterwards the obtained emulsions were stored at 70°C for at least 4 hours. After 4 hours the emulsions were analyzed for lumps. Significant amounts of lumps were found in the comparative example Com. 1. The samples according to the invention Ex.1 did not show any lumps after 4 hours storing at 70°C. Ex.1 did also not show lumps after 8 hours storing at 70°C.

### Further example formulations:

| **Ingredient** | **Ex.2** | **Ex.3** | **Ex.4** | **Ex.5** | **Ex.6** | **Ex.7** |
|---|---|---|---|---|---|---|
| Aluminum Chlorohydrate | 15 | 0 | 17 | 17 | 0 | 17 |
| Aluminum Sesquichlorohydrate | 0 | 15 | 0 | 0 | 15 | 0 |
| Palmitamidopropyltrimonium Chloride | 0.8 | 0.5 | 1 | 0.6 | 1.2 | 1.2 |
| Cetyl Palmitate | 7 | 6 | 5 | 6 | 6 | 5 |
| PPG-14 Butyl Ether | 18 | 15 | 16 | 15 | 15 | 15 |
| Hydrogenated Castor Oil | 5 | 6 | 7 | 6 | 6 | 6 |
| C12-15 Alkyl Benzoate | 7 | 5.6 | 6 | 5.6 | 5.6 | 5.6 |
| C20-40 Alkyl Stearate | 0.9 | 0.9 | 1.1 | 1 | 1.1 | 1 |
| Steareth-21 | 0.55 | 0.6 | 0.5 | 0.5 | 0.4 | 0.5 |
| Steareth-2 | 0.55 | 0.6 | 0.5 | 0.5 | 0.6 | 0.5 |
| Oleth-20 | 1.1 | 0.9 | 1 | 0.8 | 1 | 1.0 |
| Fatty Acid mixture consisting of 47% Stearic Acid, 48.5% Palmitic Acid, 2.5% Myristic Acid, 1.5% Arachidic Acid and 0.5% Oleic Acid | 4 | 3.5 | 3.8 | 3.8 | 4 | 3.8 |
| Parfume | q.s | q.s | q.s | q.s | q.s | q.s |
| Propylene Glycol | 2.0 | 2.2 | 1.5 | 2.0 | 1.0 | 2.0 |
| Cetearyl Alcohol | 3 | 2.5 | 3 | 2.5 | 2.8 | 2.5 |
| Aqua | Ad. 100 | Ad. 100 | Ad. 100 | Ad. 100 | Ad. 100 | Ad. 100 |

## Claims

1. Antiperspirant oil in water emulsion comprising, calculated to the total weight of the emulsion,
a) at least one antiperspirant active aluminum compound;
b) Palmitamidopropyltrimonium chloride;
c) at least one lipid and/or wax component with a melting point of >50° C;
d) at least one oil which is liquid at 20° C;
e) at least one nonionic emulsifier with an HLB value of more than 7;
f) at least one nonionic emulsifier with an HLB value greater than 1.0 and less than or equal to 7.0; and
g) 30% to 50% by weight of water
**characterized in that** the emulsion is liquid at 70 °C and solid at 20°C, 1013 hPa and a relative humidity of 50%.

2. Emulsion according to claim 1 **characterized in that** aluminum compound is selected from the group consisting of aluminum chlorohydrate; aluminum sesquichlorohydrate; aluminum zirconium chlorohydrates and the aluminum zirconium chlorohydrate glycine complexes, whereby aluminum chlorohydrate and/or aluminum sesquichlorohydrate are preferred.

3. Emulsion according to claim 1 or 2 **characterized in that** the total quantity of the antiperspirant active aluminum compound is in the range from 5 to 30% by weight, more preferably 10 to 25% by weight and most preferably 12.5 to 22.5% by weight, calculated to the total weight of the emulsion.

4. Emulsion according to claim 1 **characterized in that** the aluminum chlorohydrate is contained as antiperspirant active aluminum compound and the total quantity of aluminum chlorohydrate is in the range from 5 to 30% by weight, more preferably 10 to 25% by weight and most preferably 12.5 to 22.5% by weight, calculated to the total weight of the emulsion.

5. Emulsion according to claim 4 **characterized in that** no further antiperspirant active aluminum compound is contained besides aluminum chlorohydrate.

6. Emulsion according to any of the proceeding claims **characterized in that** the total quantity of palmitamidopropyltrimonium chloride is in the range from 0.1 to 5% by weight, more preferably 0.2 to 3% by weight, more preferably 0.3 to 2% by weight, more preferably 0.4 to 1.5% by weight and most preferably 0.5 to 1.2% by weight, calculated to the total weight of the emulsion.

7. Emulsion according to any proceeding claim **characterized in that** the total quantity of the lipid and/or wax components c) with a melting point of >50°C is in the range from 8 to 18% by weight, more preferably 10 to 15% by weight calculated to the total weight of the emulsion.

8. Emulsion according to any proceeding claim **characterized in that** the wax component c) is chosen from at least one ester of a saturated monohydric C16-C60-alcohol and a saturated C8-C36-monocarboxylic acid, as long as those esters have a melting point above 50°C.

9. Emulsion according to any proceeding claim **characterized in that** the wax component c) is chosen from the group of esters of saturated, branched or unbranched alkanecarboxylic acids of chain length from 12 to 24 carbon atoms and the saturated, branched or unbranched alcohols of chain length from 16 to 50 carbon atoms which have a melting point of >50° C.

10. Emulsion according to any proceeding claim **characterized in that** the total quantity of the ester of saturated, branched or unbranched alkanecarboxylic acids of chain length from 12 to 24 carbon atoms and the saturated, branched or unbranched alcohols of chain length from 16 to 50 carbon atoms, which have a melting point of >50°C, is in the range from 3 to 9% by weight, more preferably 4.5 to 7.5% by weight an most preferably, 5 to 7% by weight, calculated to the total weight of the emulsion.

11. Emulsion according to any proceeding claim **characterized in that** C20-C40-alkyl stearate is contained as wax component c), whereby it is preferred if the total quantity of C20-C40-alkyl stearate is in the range from 0.2 to 3% by weight, more preferably 0.5 to 2% by weight and most preferably 0.75 to 1.5% by weight, calculated to the total weight of the emulsion.

12. Emulsion according to any proceeding claim **characterized in that** hydrogenated castor oil is contained in the emulsion as wax component c), whereby it is preferred if the total quantity of hydrogenated castor oil is in the range from 3 to 9% by weight, more preferably 4.5 to 7.5% by weight an most preferably, 5 to 7% by weight, calculated to the total weight of the emulsion.

13. Emulsion according to any proceeding claim **characterized in that** the emulsion comprises one or more saturated linear C14-C36-carboxylic acids as wax compound c), whereby it is preferred if the total quantity of those carboxylic acid is in the range from 1 to 8% by weight, more preferably 2 to 6% by weight and most preferably 3 to 5% by weight, calculated to the total weight of the emulsion.

14. Emulsion according to any proceeding claim **characterized in that** the emulsion comprises at least one addition product of at least 6 ethylene oxide and/or propylene oxide units onto mono- or polyhydric C3-22-alkanols as oils d), whereby PPG-14 butyl ether and PPG-15 stearyl ether are preferred.

15. Emulsion according to any proceeding claim **characterized in that** the oils d) are selected from PPG-14 butyl ether, PPG-15 stearyl ether and/or C12-15 alkyl benzoate, whereby the total quantity of PPG-14 butyl ether, PPG-15 stearyl ether and/or C12-15 alkyl benzoate is in the range from 10 to 30% by weight, more preferably 15 to 27% by weight and most preferably 18 to 25% by weight, calculated to the total weight of the emulsion.

16. Emulsion according to any proceeding claim **characterized in that** the total quantity of the nonionic emulsifier e) with an HLB value of more than 7 is in the range from 0.5 to 3% by weight, more preferably 1 to 2.5% by weight, based on the total weight of the emulsion.

17. Emulsion according to any proceeding claim **characterized in that** the emulsifier e) is selected from C12-C18-alkanols having in each case 10-30 units of ethylene oxide per molecule, and mixtures of these substances.

18. Emulsion according to claim 16 or 17 **characterized in that** the emulsion comprises Steareth-21 and/or Oleth-20 as emulsifiers e) in a total quantity from 0.5 to 3% by weight, more preferably 1 to 2.5% by weight, based on the total weight of the emulsion.

19. Emulsion according to any proceeding claim **characterized in that** the at least one emulsifier e) is **characterized in that** it has an HLB value in the range from 14 to 17, more preferably from 14.5 to 16.5 and most preferably from 15 to 16.

20. Emulsion according to any proceeding claim **characterized in that** the nonionic emulsifier f) has an HLB value in the range from 3 to 6.5, more preferably 4 to 6.

21. Emulsion according to claim 20 **characterized in that** the total quantity of the emulsifier f) is in the range from 0.1% to 1.5% by weight, more preferably 0.3 to 1% by weight, calculated to the total weight of the emulsion.

22. Emulsion according to any proceeding claim **characterized in that** the emulsion comprises at least Steareth-2 as nonionic emulsifier f), whereby it is preferred if the quantity of Steareth-2 is preferably in the range from 0.1% to 1.5% by weight, more preferably 0.3 to 1% by weight, calculated to the total weight of the emulsion.

23. Emulsion according to any of the proceeding claims **characterized in that** the emulsion comprises cetyl alcohol, stearyl alcohol and/or mixtures thereof, whereby it is preferred, if the total quantity of those alcohols is in the range from 1 to 5% and more preferably from 1.5% to 3.5% by weight calculated to the total weight of the emulsion.

## Patentansprüche

1. Antitranspirant-Öl-in-Wasser-Emulsion, umfassend, berechnet auf das Gesamtgewicht der Emulsion,
a) mindestens eine antitranspirant wirksame Aluminiumverbindung;
b) Palmitamidopropyltrimoniumchlorid;
c) mindestens eine Lipid- und/oder Wachskomponente mit einem Schmelzpunkt >50 °C;
d) mindestens ein bei 20 °C flüssiges Öl;
e) mindestens einen nichtionischen Emulgator mit einem HLB-Wert von mehr als 7;
f) mindestens einen nichtionischen Emulgator mit einem HLB-Wert größer 1,0 und kleiner oder gleich 7,0; und
g) 30 bis 50 Gew.-% Wasser,
**dadurch gekennzeichnet, dass** die Emulsion bei 70 °C flüssig und bei 20 °C, 1013 hPa und einer relativen Luftfeuchte von 50 % fest ist.

2. Emulsion nach Anspruch 1, **dadurch gekennzeichnet, dass** die Aluminiumverbindung aus der Gruppe bestehend aus Aluminiumchlorhydrat, Aluminiumsesquichlorhydrat, Aluminiumzirconiumchlorhydraten und den Aluminiumzirconiumchlorhydrat-Glycin-Komplexen ausgewählt ist, wobei Aluminiumchlorhydrat und/oder Aluminiumsesquichlorhydrat bevorzugt sind.

3. Emulsion nach Anspruch 1 oder 2, **dadurch gekennzeichnet, dass** die Gesamtmenge der antitranspirant wirksamen Aluminiumverbindung im Bereich von 5 bis 30 Gew.-%, weiter bevorzugt 10 bis 25 Gew.-% und ganz besonders bevorzugt 12,5 bis 22,5 Gew.-%, berechnet auf das Gesamtgewicht der Emulsion, liegt.

4. Emulsion nach Anspruch 1, **dadurch gekennzeichnet, dass** das Aluminiumchlorhydrat als antitranspirant wirksame Aluminiumverbindung enthalten ist und die Gesamtmenge von Aluminiumchlorhydrat im Bereich von 5 bis 30 Gew.-%, weiter bevorzugt 10 bis 25 Gew.-% und ganz besonders bevorzugt 12,5 bis 22,5 Gew.-%, berechnet auf das Gesamtgewicht der Emulsion, liegt.

5. Emulsion nach Anspruch 4, **dadurch gekennzeichnet, dass** neben Aluminiumchlorhydrat keine weitere antitranspirant wirksame Aluminiumverbindung enthalten ist.

6. Emulsion nach einem der vorhergehenden Ansprüche, **dadurch gekennzeichnet, dass** die Gesamtmenge von Palmitamidopropyltrimoniumchlorid im Bereich von 0,1 bis 5 Gew.-%, weiter bevorzugt 0,2 bis 3 Gew.-%, weiter bevorzugt 0,3 bis 2 Gew.-%, weiter bevorzugt 0,4 bis 1,5 Gew.-% und ganz besonders bevorzugt 0,5 bis 1,2 Gew.-%, berechnet auf das Gesamtgewicht der Emulsion, liegt.

7. Emulsion nach einem der vorhergehenden Ansprüche, **dadurch gekennzeichnet, dass** die Gesamtmenge der Lipid- und/oder Wachskomponenten c) mit einem Schmelzpunkt >50 °C im Bereich von 8 bis 18 Gew.-%, weiter bevorzugt 10 bis 15 Gew.-%, berechnet auf das Gesamtgewicht der Emulsion, liegt.

8. Emulsion nach einem der vorhergehenden Ansprüche, **dadurch gekennzeichnet, dass** die Wachskomponente c) aus mindestens einem Ester eines gesättigten einwertigen C16-C60-Alkohols und einer gesättigten C8-C36-Monocarbonsäure ausgewählt ist, solange diese Ester einen Schmelzpunkt über 50 °C aufweisen.

9. Emulsion nach einem der vorhergehenden Ansprüche, **dadurch gekennzeichnet, dass** die Wachskomponente c) aus der Gruppe von Estern von gesättigten, verzweigten oder unverzweigten Alkancarbonsäuren mit einer Kettenlänge von 12 bis 24 Kohlenstoffatomen und den gesättigten, verzweigten oder unverzweigten Alkoholen mit einer Kettenlänge von 16 bis 50 Kohlenstoffatomen, die einen Schmelzpunkt >50 °C aufweisen, ausgewählt ist.

10. Emulsion nach einem der vorhergehenden Ansprüche, **dadurch gekennzeichnet, dass** die Gesamtmenge der Ester von gesättigten, verzweigten oder unverzweigten Alkancarbonsäuren mit einer Kettenlänge von 12 bis 24 Kohlenstoffatomen und den gesättigten, verzweigten oder unverzweigten Alkoholen mit einer Kettenlänge von 16 bis 50 Kohlenstoffatomen, die einen Schmelzpunkt >50 °C aufweisen, im Bereich von 3 bis 9 Gew.-%, weiter bevorzugt 4,5 bis 7,5 Gew.-% und ganz besonders bevorzugt 5 bis 7 Gew.-%, berechnet auf das Gesamtgewicht der Emulsion, liegt.

11. Emulsion nach einem der vorhergehenden Ansprüche, **dadurch gekennzeichnet, dass** C20-C40-Alkylstearat als Wachskomponente c) enthalten ist, wobei es bevorzugt ist, wenn die Gesamtmenge von C20-C40-Alkylstearat im Bereich von 0,2 bis 3 Gew.-%, weiter bevorzugt 0,5 bis 2 Gew.-% und ganz besonders bevorzugt 0,75 bis 1,5 Gew.-%, berechnet auf das Gesamtgewicht der Emulsion, liegt.

12. Emulsion nach einem der vorhergehenden Ansprüche, **dadurch gekennzeichnet, dass** hydriertes Rizinusöl in der Emulsion als Wachskomponente c) enthalten ist, wobei es bevorzugt ist, wenn die Gesamtmenge von hydriertem Rizinusöl im Bereich von 3 bis 9 Gew.-%, weiter bevorzugt 4,5 bis 7,5 Gew.-% und besonders bevorzugt 5 bis 7 Gew.- %, berechnet auf das Gesamtgewicht der Emulsion, liegt.

13. Emulsion nach einem der vorhergehenden Ansprüche, **dadurch gekennzeichnet, dass** die Emulsion eine oder mehrere gesättigte lineare C14-C36-Carbonsäuren als Wachskomponente c) umfasst, wobei es bevorzugt ist, wenn die Gesamtmenge dieser Carbonsäure im Bereich von 1 bis 8 Gew.-%, weiter bevorzugt 2 bis 6 Gew.-% und ganz besonders bevorzugt 3 bis 5 Gew.-%, berechnet auf das Gesamtgewicht der Emulsion, liegt.

14. Emulsion nach einem der vorhergehenden Ansprüche, **dadurch gekennzeichnet, dass** die Emulsion mindestens ein Anlagerungsprodukt von mindestens 6 Ethylenoxid- und/oder Propylenoxid-Einheiten an ein- oder mehrwertige C3-22-Alkanole als Öle d) umfasst, wobei PPG-14-Butylether und PPG-15-Stearylether bevorzugt sind.

15. Emulsion nach einem der vorhergehenden Ansprüche, **dadurch gekennzeichnet, dass** die Öle d) aus PPG-14-Butylether, PPG-15-Stearylether und/oder C12-15-Alkylbenzoat ausgewählt sind, wobei die Gesamtmenge von PPG-14-Butylether, PPG-15-Stearylether und/oder C12-15-Alkylbenzoat im Bereich von 10 bis 30 Gew.-%, weiter bevorzugt 15 bis 27 Gew.-% und ganz besonders bevorzugt 18 bis 25 Gew.-%, berechnet auf das Gesamtgewicht der Emulsion, liegt.

16. Emulsion nach einem der vorhergehenden Ansprüche, **dadurch gekennzeichnet, dass** die Gesamtmenge des nichtionischen Emulgators e) mit einem HLB-Wert von mehr als 7 im Bereich von 0,5 bis 3 Gew.-%, weiter bevorzugt 1 bis 2,5 Gew.-%, bezogen auf das Gesamtgewicht der Emulsion, liegt.

17. Emulsion nach einem der vorhergehenden Ansprüche, **dadurch gekennzeichnet, dass** der Emulgator e) aus C12-C18-Alkanolen mit jeweils 10-30 Einheiten Ethylenoxid pro Molekül und Mischungen dieser Substanzen ausgewählt ist.

18. Emulsion nach Anspruch 16 oder 17, **dadurch gekennzeichnet, dass** die Emulsion Steareth-21 und/oder Oleth-20 als Emulgatoren e) in einer Gesamtmenge von 0,5 bis 3 Gew.-%, weiter bevorzugt 1 bis 2,5 Gew.-%, bezogen auf das Gesamtgewicht der Emulsion, umfasst.

19. Emulsion nach einem der vorhergehenden Ansprüche, **dadurch gekennzeichnet, dass** der mindestens eine Emulgator e) **dadurch gekennzeichnet ist, dass** er einen HLB-Wert im Bereich von 14 bis 17, weiter bevorzugt von 40,5 bis 16,5 und ganz besonders bevorzugt von 15 bis 16 aufweist.

20. Emulsion nach einem der vorhergehenden Ansprüche, **dadurch gekennzeichnet, dass** der nichtionische Emulgator f) einen HLB-Wert im Bereich von 3 bis 6,5, weiter bevorzugt 4 bis 6, aufweist.

21. Emulsion nach Anspruch 20, **dadurch gekennzeichnet, dass** die Gesamtmenge des Emulgators f) im Bereich von 0,1 bis 1,5 Gew.-%, weiter bevorzugt 0,3 bis 1 Gew.-%, berechnet auf das Gesamtgewicht der Emulsion, liegt.

22. Emulsion nach einem der vorhergehenden Ansprüche, **dadurch gekennzeichnet, dass** die Emulsion zumindest Steareth-2 als nichtionischen Emulgator f) umfasst, wobei es bevorzugt ist, wenn die Menge von Steareth-2 vorzugsweise im Bereich von 0,1 bis 1,5 Gew.-%, weiter bevorzugt 0,3 bis 1 Gew.-%, berechnet auf das Gesamtgewicht der Emulsion, liegt.

23. Emulsion nach einem der vorhergehenden Ansprüche, **dadurch gekennzeichnet, dass** die Emulsion Cetylalkohol, Stearylalkohol und/oder Mischungen davon umfasst, wobei es bevorzugt ist, wenn die Gesamtmenge dieser Alkohol im Bereich von 1 bis 5 Gew.-% und weiter bevorzugt 1,5 bis 3,5 Gew.-%, berechnet auf das Gesamtgewicht der Emulsion, liegt.

## Revendications

1. Émulsion antitranspirante huile dans eau comprenant, calculé par rapport au poids total de l'émulsion,
a) au moins un composé d'aluminium actif antitranspirant ;
b) du chlorure de palmitamidopropyltrimonium ;
c) au moins un composant lipide et/ou cire ayant un point de fusion > 50 °C ;
d) au moins une huile qui est liquide à 20 °C ;
e) au moins un émulsifiant non ionique ayant une valeur HLB supérieure à 7 ;
f) au moins un émulsifiant non ionique ayant une valeur HLB supérieure à 1,0 et inférieure ou égale à 7,0 ; et
g) 30 % à 50 % en poids d'eau
**caractérisée en ce que** l'émulsion est liquide à 70 °C et solide à 20 °C, 1 013 hPa et une humidité relative de 50 %.

2. Émulsion selon la revendication 1, **caractérisée en ce que** le composé d'aluminium est choisi dans le groupe constitué par le chlorhydrate d'aluminium ; le sesquichlorohydrate d'aluminium ; les chlorhydrates d'aluminium zirconium et les complexes glycine chlorhydrate d'aluminium zirconium, le chlorhydrate d'aluminium et/ou le sesquichlorohydrate d'aluminium étant préférés.

3. Émulsion selon la revendication 1 ou 2, **caractérisée en ce que** la quantité totale du composé d'aluminium actif antitranspirant est dans la plage de 5 à 30 % en poids, plus préférablement de 10 à 25 % en poids et le plus préférablement de 12,5 à 22,5 % en poids, calculée par rapport au poids total de l'émulsion.

4. Émulsion selon la revendication 1, **caractérisée en ce que** le chlorhydrate d'aluminium est contenu en tant que composé d'aluminium actif antitranspirant et la quantité totale de chlorhydrate d'aluminium est dans la plage de 5 à 30 % en poids, plus préférablement de 10 à 25 % en poids et le plus préférablement de 12,5 à 22,5 % en poids, calculée par rapport au poids total de l'émulsion.

5. Émulsion selon la revendication 4, **caractérisée en ce qu'**aucun autre composé d'aluminium actif antitranspirant n'est contenu outre le chlorhydrate d'aluminium.

6. Émulsion selon l'une quelconque des revendications précédentes, **caractérisée en ce que** la quantité totale de chlorure de palmitamidopropyltrimonium est dans la plage de 0,1 à 5 % en poids, plus préférablement de 0,2 à 3 % en poids, plus préférablement de 0,3 à 2 % en poids, plus préférablement de 0,4 à 1,5 % en poids et le plus préférablement de 0,5 à 1,2 % en poids, calculée par rapport au poids total de l'émulsion.

7. Émulsion selon l'une quelconque des revendications précédentes, **caractérisée en ce que** la quantité totale des composants lipide et/ou cire c) ayant un point de fusion > 50 °C est dans la plage de 8 à 18 % en poids, plus préférablement de 10 à 15 % en poids, calculée par rapport au poids total de l'émulsion.

8. Émulsion selon l'une quelconque des revendications précédentes, **caractérisée en ce que** le composant cire c) est choisi parmi au moins un ester d'un alcool monohydrique saturé en C16-C60 et d'un acide monocarboxylique saturé en C8-C36, pour autant que ces esters aient un point de fusion supérieur à 50 °C.

9. Émulsion selon l'une quelconque des revendications précédentes, **caractérisée en ce que** le composant cire c) est choisi dans le groupe des esters d'acides alcanecarboxyliques saturés, ramifiés ou non ramifiés ayant une longueur de chaîne de 12 à 24 atomes de carbone et des alcools saturés, ramifiés ou non ramifiés ayant une longueur de chaîne de 16 à 50 atomes de carbone qui ont un point de fusion > 50 °C.

10. Émulsion selon l'une quelconque des revendications précédentes, **caractérisée en ce que** la quantité totale de l'ester d'acides alcanecarboxyliques saturés, ramifiés ou non ramifiés ayant une longueur de chaîne de 12 à 24 atomes de carbone et des alcools saturés, ramifiés ou non ramifiés ayant une longueur de chaîne de 16 à 50 atomes de carbone, qui ont un point de fusion > 50 °C, est dans la plage de 3 à 9 % en poids, plus préférablement de 4,5 à 7,5 % en poids et le plus préférablement de 5 à 7 % en poids, calculée par rapport au poids total de l'émulsion.

11. Émulsion selon l'une quelconque des revendications précédentes, **caractérisée en ce que** du stéarate d'alkyle en C20-C40 est contenu en tant que composant cire c), étant préféré que la quantité totale de stéarate d'alkyle en C20-C40 se situe dans la plage de 0,2 à 3 % en poids, plus préférablement de 0,5 à 2 % en poids et le plus préférablement de 0,75 à 1,5 % en poids, calculée par rapport au poids total de l'émulsion.

12. Émulsion selon l'une quelconque des revendications précédentes, **caractérisée en ce que** de l'huile de ricin hydrogénée est contenue dans l'émulsion en tant que composant cire c), étant préféré que la quantité totale d'huile de ricin hydrogénée se situe dans la plage de 3 à 9 % en poids, plus préférablement de 4,5 à 7,5 % en poids et le plus préférablement de 5 à 7 % en poids, calculée par rapport au poids total de l'émulsion.

13. Émulsion selon l'une quelconque des revendications précédentes, **caractérisée en ce que** l'émulsion comprend un ou plusieurs acides carboxyliques linéaires saturés en C14-C36 en tant que composé cire c), étant préféré que la quantité totale de ces acides carboxyliques se situe dans la plage de 1 à 8 % en poids, plus préférablement de 2 à 6 % en poids et le plus préférablement de 3 à 5 % en poids, calculée par rapport au poids total de l'émulsion.

14. Émulsion selon l'une quelconque des revendications précédentes, **caractérisée en ce que** l'émulsion comprend au moins un produit d'addition d'au moins 6 motifs d'oxyde d'éthylène et/ou oxyde de propylène sur des alcanols en C3-22 mono- ou polyhydriques en tant qu'huiles d), l'éther de butyle de PPG-14 et l'éther de stéaryle de PPG-15 étant préférés.

15. Émulsion selon l'une quelconque des revendications précédentes, **caractérisée en ce que** les huiles d) sont choisies parmi l'éther de butyle de PPG-14, l'éther de stéaryle de PPG-15 et/ou un benzoate d'alkyle en C12-15, la quantité totale d'éther de butyle de PPG-14, d'éther de stéaryle de PPG-15 et/ou de benzoate d'alkyle en C12-15 étant dans la plage de 10 à 30 % en poids, plus préférablement de 15 à 27 % en poids et le plus préférablement de 18 à 25 % en poids, calculée par rapport au poids total de l'émulsion.

16. Émulsion selon l'une quelconque des revendications précédentes, **caractérisée en ce que** la quantité totale de l'émulsifiant non ionique e) ayant une valeur HLB supérieure à 7 est dans la plage de 0,5 à 3 % en poids, plus préférablement de 1 à 2,5 % en poids, par rapport au poids total de l'émulsion.

17. Émulsion selon l'une quelconque des revendications précédentes, **caractérisée en ce que** l'émulsifiant e) est choisi parmi des alcanols en C12-C18 ayant en chaque cas 10-30 motifs d'oxyde d'éthylène par molécule, et des mélanges de ces substances.

18. Émulsion selon la revendication 16 ou 17, **caractérisée en ce que** l'émulsion comprend du Stéareth-21 et/ou de l'Oleth-20 en tant qu'émulsifiants e) en une quantité totale de 0,5 à 3 % en poids, plus préférablement de 1 à 2,5 % en poids, par rapport au poids total de l'émulsion.

19. Émulsion selon l'une quelconque des revendications précédentes, **caractérisée en ce que** l'au moins un émulsifiant e) est **caractérisé en ce qu'**il a une valeur HLB dans la plage de 14 à 17, plus préférablement de 14,5 à 16,5 et le plus préférablement de 15 à 16.

20. Émulsion selon l'une quelconque des revendications précédentes, **caractérisée en ce que** l'émulsifiant non ionique f) a une valeur HLB dans la plage de 3 à 6,5, plus préférablement de 4 à 6.

21. Émulsion selon la revendication 20, **caractérisée en ce que** la quantité totale de l'émulsifiant f) est dans la plage de 0,1 % à 1,5 % en poids, plus préférablement de 0,3 % à 1 % en poids, calculée par rapport au poids total de l'émulsion.

22. Émulsion selon l'une quelconque des revendications précédentes, **caractérisée en ce que** l'émulsion comprend au moins du Stéareth-2 en tant qu'émulsifiant non ionique f), étant préféré que la quantité de Stéareth-2 soit de préférence dans la plage de 0,1 % à 1,5 % en poids, plus préférablement de 0,3 à 1 % en poids, calculée par rapport au poids total de l'émulsion.

23. Émulsion selon l'une quelconque des revendications précédentes, **caractérisée en ce que** l'émulsion comprend de l'alcool cétylique, de l'alcool stéarylique et/ou des mélanges de ceux-ci, étant préféré que la quantité totale de ces alcools se situe dans la plage de 1 à 5 % et plus préférablement de 1,5 à 3,5 % en poids, calculée par rapport au poids total de l'émulsion.
